# EUROPEAN PATENT SPECIFICATION

(11) **EP 3 471 541 B1**
(45) Date of publication and mention of the grant of the patent: **05.05.2021**
(21) Application number: 17734842.2
(22) Date of filing: 19.06.2017
(51) Int. Cl.: A61K 31/23, A61P 31/00, A01N 31/02, A01N 37/02, A01N 37/06, A61P 31/04, A61P 31/10, A61P 31/12, A01P 1/00

(54) **SYNERGISTIC EFFECTS, AUGMENTING ANTIMICROBIAL EFFECTS OF LIPIDS**
SYNERGISTISCHE WIRKUNGEN, DIE DIE ANTIMIKROBIELLEN WIRKUNGEN VON LIPIDEN VERSTÄRKEN
EFFETS SYNERGIQUES AUGMENTANT LES EFFETS ANTIMICROBIENS DES LIPIDES

(30) Priority: 20.06.2016 IS 50152
(43) Date of publication of application: 24.04.2019
(73) Proprietor: Capretto EHF., 610 Grenivik (IS)
(72) Inventor: GIZURARSON, Sveinbjorn, 107 Reykjavik (IS); HELGADOTTIR, Helga, 201 Kópavogur (IS); KRISTMUNDSDOTTIR, Thordis, 170 Seltjarnarnes (IS); THORMAR, Halldor, 108 Reykjavik (IS); GUDMUNDSSON, Sigurdur, 200 Kopavogur (IS)
(74) Representative: Arnason Faktor
(86) International application number: PCT/IS2017/050010
(87) International publication number: WO 2017/221276

(56) References cited:
- US-A1- 2005 043 402
- OH D-H ET AL: "Antimicrobial activity of ethanol, glycerol monolaurate or lactic acid against Listeria monocytogenes", INTERNATIONAL JOURNAL OF FOOD MICROBIOLOGY, ELSEVIER BV, NL, vol. 20, no. 4, 1 December 1993 (1993-12-01), pages 239-246, XP023697718, ISSN: 0168-1605, DOI: 10.1016/0168-1605(93)90168-G [retrieved on 1993-12-01]
- CHIFU B HUANG ET AL: "Short- and medium-chain fatty acids exhibit antimicrobial activity for oral microorganisms", ARCHIVES OF ORAL BIOLOGY, PERGAMON PRESS, OXFORD, GB, vol. 56, no. 7, 20 January 2011 (2011-01-20), pages 650-654, XP028378340, ISSN: 0003-9969, DOI: 10.1016/J.ARCHORALBIO.2011.01.011 [retrieved on 2011-02-01]

## Description

### Field

This invention relates to novel formulations with microbicidal lipids, comprising ethanol, which enhances the activity of the microbicidal lipid.

### Introduction

There are several published reports on antiviral and antibacterial activities of milk lipids (J. K. Welsh et al. Use of Semliki Forest virus to identify lipid-mediated antiviral activity and anti-alphavirus immunoglobulin A in human milk, Infect. Immun. 19, 395-401, 1978; J. K. Welsh et al. Effect of antiviral lipids, heat and freezing on the activity of viruses in human milk, J. Infect. Dis. 140,322-328,1979; J. J. Kabara, Fatty acids and derivatives as antimicrobial agents. In: The pharmacological effect of lipids. Edited by J. J. Kabara. The American Oil Chemists Society, St. Louis, Mo., 1978, pp. 1-13; C. E. Isaacs, H. Thormar et al., Membrane disruptive effect of human milk: Inactivation of enveloped viruses, J. Infect. Dis. 154, 966-971, 1986; C. E. Isaacs and H. Thormar, Human milk lipids inactivate enveloped viruses. In: Breastfeeding, Nutrition, Infection and Infant Growth in Developed and Emerging Countries. Edited by S. A. Atkinson, L. A. Hanson, R. K. Chandra. ARTS Biomedical Publ. St. Johns, Newfoundland, Canada. 1990, pp. 161-174; C. E. Isaacs et al., Antiviral and antibacterial lipids in human milk and infant formula feeds, Arch. Dis. Childhood 65, 861-864, 1990; C. E. Isaacs and H. Thormar, The role of milk-derived antimicrobial lipids as antiviral and antibacterial agents. In: Immunology of Milk and the Neonate. Edited by J. Mestecky et al. Plenum Press, 1991, pp. 159-165; C. E. Isaacs et al., Addition of lipases to infant formulas produces antiviral and antibacterial activity, J. Nutr. Biochem. 3, 304-308, 1992; C. E. Isaacs et al. Antimicrobial activity of lipids added to human milk, infant formula, and bovine milk, Nutr. Biochem. 6, 362-366, 1995) where the active lipids are free fatty acids and monoglycerides which are released from triglycerides in the milk by milk lipases or lipases of the gastrointestinal tract.

The virucidal effect of purified lipids has been studied in cell culture media (H. Thormar, C. E. Isaacs et al., Inactivation of enveloped viruses and killing of cells by fatty acids and monoglycerides. Antimicr. Agents Chemother. 31, 27-31, 1987; H. Thormar, C. E. Isaacs et. al., Inactivation of visna virus and other enveloped viruses by free fatty acids and monoglycerides. Ann. N.Y. Acad. Sci. 724, 465-471, 1994).

Enveloped viruses, such as herpes simplex virus type 1 (HSV-1), vesicular stomatitis virus (VSV) and visna virus, were found to be inactivated by log-chain unsaturated and medium-chain saturated fatty acids, whereas long-chain saturated and short-chain fatty acids had no or only a very small virucidal effect at the highest concentrations tested. 1-monoglycerides of medium-chain unsaturated fatty acids showed more virucidal activity than the corresponding free fatty acids. Thus, capric acid 1-monoglyceride (10:0) capric acid 1-monoglyceride is also denoted monocaprin or MC in the following) and lauric acid 1-monoglyceride (12:0) were 10-fold more active than capric and lauric acids (by the designation (X:Y) is meant that a fatty moiety consists of X carbon atoms and comprises Y double bonds). Capric acid 1-monoglyceride at a concentration of 2 mM and lauric acid 1-monoglyceride at a concentration of 1 mM caused a 3000-fold to 10,000-fold reduction in titer of HSV-1, VSV and visna virus when incubated in cell culture medium at 37°C. for 30 min. Diglycerides of fatty acids showed no virucidal activity. An electron microscope study, using the negative staining technique showed that virucidal fatty acids caused leakage of the viral envelope of VSV and at a higher concentration a complete disintegration of the envelope and the viral particles. They also caused disintegration of the plasma membranes of tissue culture cells resulting in cell lysis and death (H. Thormar et al. Antimicrob. Agents Chemother. 31, 27-31, 1987). The mechanism of disruption of cellular and viral membranes by lipids is not known.

The microbicidal and cytocidal activities of lipids and their potential applications for killing microorganisms in bodily fluids are described in the U.S. Pat. No. 4,997,851 and 5,434,182. Their application for disinfecting contact lenses is described in U.S. Pat. No. 5,624,958 and their application for counteracting infection of mucosa or skin in a patent application No.: 10/408235.

US 2005/043402 relates to a method for preventing infection of the genital mucosa of a mammal by virus, pathogenic bacteria or fungi, comprising topically administering, to the genital mucosa an effective prophylactic amount of a formulation comprising a hydrogel which comprises a microbicidal lipid as an active ingredient, a water gelling agent and a solubilizing agent. The microbicidal lipid is preferably a C6-18 fatty acid or salt thereof, fatty acid monoglycerides, fatty acid esters of monohydric alcohols, fatty alcohols or fatty alcohol monoglycerides ether.

### Summary of the Invention

This invention is based on inventors' finding of a surprising and substantial synergistic effect, where the antimicrobial effects of microbicidal lipids defined in the claims may be augmented with minor amounts of ethanol. The invention relates in particular to augmenting the microbicidal effects for preventing infection in the nasal, ocular, otal, pharynx, larynx, sinuses, oral cavity, vaginal or dermal surface of a mammal by virus, pathogenic bacteria or fungi.

According to the present invention, it has been found that small amounts of ethanol, within the range 0,2-4% and more preferably in the range 0,5-3% had surprising effects of the antimicrobial effects induced by microbicidal lipids as defined in the claims. The formulation containing ethanol (ethyl alcohol) and lipids together had surprisingly powerful microbicidal effects when combined with microbicidal lipids defined in the claims used to treat or prevent infections infection in the nasal, ocular, otal, pharynx, larynx, sinuses, oral cavity, vaginal or dermal surface of a mammal, caused by virus, pathogenic bacteria or fungi.

The particular formulation or composition (in the present context, these terms are synonymous) contains the microbicidal lipid dissolved in a concentration in the range from 0.01 to 5% in a formulation together with 0,2-4%, preferably 0,5-3% ethanol at a concentration within the range of 0,2-5%, preferably in the range 0,5-3%, allowing the lipid to exert its killing effect in the aqueous environment prevailing at mammalian surfaces such as the nasal, ocular, otal, pharynx, larynx, sinuses, oral cavity, vaginal or dermal surface.

A further aspect of the invention relates to a lipid as defined in the claims for use as a medicament for treating infections caused by virus, bacteria or fungi in skin or mucosal membranes, in particular infection in the nasal, ocular, otal, pharynx, larynx, sinuses, oral cavity, vaginal or dermal surface, comprising administering an effective amount of the formulation which contains at least one microbicidal lipid as defined in the claims as an active ingredient.

### Detailed description of the invention

The term "microbicidal lipid" is used herein to designate a lipid, which is capable of killing viruses and/or bacteria and/or fungi. As explained above, such lipids have been known for some time, and have been found also to have cytocidal effect. Killing of a virus means that the virus, upon effective exposure to the lipid, will be unable to infect cells, i.e. the virus will be unable to introduce its genetic material into a cell wherein the genetic material can be reproduced. Killing of a bacterium or a cell means that the bacterium or the cell, upon effective exposure to the lipid, is no longer capable of performing the basic functions of life; particularly, the bacterium or the cell will no longer be able to obtain the necessary nutrition in order to maintain the physical integrity of the cell. As is seen from the claims and explanations given herein, it is contemplated, and considered justified to assume, that the lipids also analogously have a potent fungicidal effect.

The formulation of the invention is generally a liquid, semi-liquid or solid formulation. The presently preferred formulation comprises a solution, emulsions, suspensions, viscous fluid, semisolid such as but not limited to a gel or gel-like composition, in particular a hydrogel, or suppositories or vagitories, that can be applied to and remain in contact with the nasal, ocular, otal, pharynx, larynx, sinuses, oral cavity, vaginal or dermal surface of a mammal. For many mucosal surfaces, an aqueous solution is useful.

The aqueous solution in the formulation is preferably provided by having water as a constituent, normally a major constituent, of the formulation, but it is contemplated that the aqueous solution may also in certain cases be provided by various excipients such as but not limited to solubilizers, bioadhesive agents and surfactants.

The ethanol (ethyl alcohol) is added to a formulation containing the antimicrobial lipids or glycerides as component(s) present in an amount of at least 0.01 wt% or into which the antimicrobial lipids or glycerides are to be added. The ethanol is present in the final formulation in a concentration within a range from 0,2%, such as from 0,3%, such as from 0,4%, such as from
0,5%, to 4%, such as to 3%, such as to 2,5%, such as, but not limited to, 0,2%, 0,3%, 0,4%, 0,5%, 0,8%, 1,0%, 1,2%, 1,5%, 1,7%, 2%, 2,25, and 2,5%.

Unless otherwise specified, all weight percents are weight/weight percentages based on the total weight of a "ready to use" or "as used" composition.

Preferably, the antimicrobial lipid component comprises a monoester of a C₈-C₁₂ fatty acid(s), in position 1, having either S- isomer or R-isomer or combination thereof. Monoester having the fatty acid in position 2 is also used according to the invention and the lipid may additionally comprise a minor amount of diglycerides, triglycerides, pure glycerol and pure fatty acid.

The pharmaceutical composition containing ethanol (ethyl alcohol) of the invention comprises a biologically active lipid selected from the group consisting of but not limited to glycerol monocaprate, glycerol monocaprylate, glycerol monolaurate, propylene glycol monocaprate, propylene glycol monocaprylate, glycerol dicaprin, glycerol dicaprylate, glycerol dilaurate, glycerol tricaprin, glycerol tricaprylate, glycerol trilaurate, octylglycerol, monomyristin, monopalmitolein, monoolein, propylene glycol monolaurate, coca butter, and combinations thereof

The active lipid ingredient, such as any of the above mentioned, is typically and preferably in a concentration within a range from 0.01%, such as from 0,05%, such as from 0,1%, such as from 0,2%, to 5%, such as to 2,5% or 2%, such as to 1,5% such as to 1%. The selected concentration may depend on the intended delivery form (solution, spray, gel, etc.) and intended location of application, as further described herein. In some embodiments the lipid substance is present in a concentration of 0,1%, or 0,2%, or 0,25%, or 0,3%, or 0,4%, or 0,5%, or 0,6% or 0,7% or 0,8%, or 0,9% or 1,0%.

The antimicrobial lipids and the formulations of the present invention are suitably used to kill one or more of the following viruses (but not limited to those): herpes virus type 1 and herpes virus type 2 (HSV-2), HIV, respiratory syncytial virus (RSV), influenza A virus and parainfluenza virus type 2, Adenoviruses, Coronavirus, Rhinovirus, Enterovirus, Human metapneumovirus, Varicella zoster virus, Zika virus; also one or more of but not limited to the following bacteria *Staphylococcus aureus, Staphylococcus epidermis, Streptococci A, Streptococci pyogenes, Haemophilus influenzae, Streptococcus D, Streptococcus mutans, Streptococcus pneumoniae, Corynebacteria* sp. *Nococardia asteroides, Micrococcus sp. Pseudomonas aeruginosa, Listeria monocytogenes, Lactobacillus jensenii, Chlamydia trachomatis, Neisseria gonorrhoeae, Helicobacter pylori, Campylobacter jejuni, Mycobacterium tuberculosis, Moraxella catarrhalis, Veillonella parvula, Klebsiella species, Bordetella pertussis, Bordetella bronchiseptica, Corynebacterium diphtheria, Bacillus anthracis;* following fungi but not limited to *Candida albicans, C. albicans, C. tripicalis, C. parapsilosis, C. glabrata, C. parakrusei, C. guillermondi, C. dubliniensis, Trichophyton rubrum, Malassezia*; and/or one or more of but not limited to the following prions: "mad cow" disease.

Of course, salts, metabolic precursors, derivatives and mixtures of these antibacterial lipids may also be used where desired.

As has already been pointed out the active substance is in certain embodiments, depending on the intended use and application site, present in an effective amount within a total volume of less than 10 mL, preferably less than 1000 µL, for certain surfaces the total volume of less than 500 µL are preferable, or more preferably within the range 50-150 µL. Accordingly, the formulation of the invention is in some embodiments provided in a dosage unit providing a unit dose within the above mentioned ranges and amounts.

The pharmaceutical preparation of the invention may furthermore comprise pharmaceutically acceptable excipients, such as but not limited to methoxy-polyethyleneglycol (e.g. mPEG 350), appropriate for each delivery route or site, preferably in a concentration within a range from 0.0001%, such as from 0,01%, such as from 0,1%, to 99%, such as to 95%, such as to 90%, such as to 80%, such as to 75%, such as to 70%, such as to 60%, such as to 50%, such as to 40%, such as to 20%, such as to 15%, such as to 10%, such as to 5%, such as to 4%, or to 3%, or to 2%, such as but not limited to 1%, 1,5%, 2%, 2,5%, 3%, or 3,5%.

In some embodiments the pharmaceutical preparation additionally comprises minor proportions of one or more substance(s) selected from the group consisting of absorption promoters, water absorbing polymers, microspheres, oils, emulsions, liposomes, substances that inhibit enzymatic degradation, alcohols, organic solvents, water, surfactants, hydrophobic agents, pH-controlling agents, preservatives and osmotic pressure controlling agents, cyclodextrins and propellants or mixtures thereof.

Methoxypolyethylene glycol as used herein refers generally to polyethylene glycol polymers, with a terminal methyl group. The term can be appbreviated as mPEG. As shown in Formula (I), the methoxypolyethylene glycol substance used in the present invention has polymer chain length with n being an integer in the range from 1 to 25, such as within a range from 2, or from 3, or from 4, to 25, such as to 22, or to 20, or to 15, or to 12, or to 10. The mPEG may have a relatively uniform polymer length or a distribution of chains of different polymer length, within the given range. In some embodiments the distribution has a preferred average molecular weight, such as 350, 450, 550 or 650, corresponding to n being the average of 7.2, 9.5, 11,7, and 14, respectively. In one embodiment a combination product used in the formulation containing one or more substance(s) represented in the formula I is methoxypolyethylene glycol 350 (mPEG 350, such as Carbovax™ (DOW Chemical Company) and in another embodiment a combination used is the methoxypolyethylene glycol 550 (mPEG 550, e.g. Carbovax™). The numbers 350 and 550 refer respectively to average molecular weight of the respective substance.

Especially preferred for use in vehicle compositions according to the invention is Carbovax™ Sentry™ (mPEG 350 and mPEG 550) which refers to commercially available solvents of polymers of the above formula I, wherein n is mainly x and y, respectively, manufactured by The Dow Chemical Company mPEG 350 and mPEG 550 are colourless liquid miscible with water, alcohols, such as methanol, ethanol, n-proypanol, glycerol and various oils in all proportions and has a b.p. about 155°C. Both mPEG 350 and mPEG 550 are reported to be non-irritating when used in compositions for parenteral administration undiluted form as stated by Dow Chemicals.

The methoxypolyethylene glycols used in accordance with the present invention may e.g. be methoxy-diethyleneglyol (m2EG), methoxy-triethylene glycol (m3EG), methoxy-tetraethylene glycol (m4EG), methoxy-pentaethylene glycol (m5EG), methoxy-hexaethylene glycol (m6EG), methoxy-heptaethylene glycol (m7EG), methoxy-octaethylene glycol (m8EG), methoxy-nonaethylene glycol (m9EG), methoxy-decaethylene glycol (m10EG), methoxy-undecaethylene glycol (m11EG), methoxy-dodecaethylene glycol (m12EG), methoxy-tridecaethylene glycol (m13EG) and methoxy-tetradecaethylene glycol (m14EG). The ethylene glycols may be used in the form of the single compounds or a mixture of two or more methoxy-n-ethylene glycols, e.g. commercial products such as Carbovax™ Sentry™ (mPEG 350 or mPEG 550).

Methoxypolyethylene glycols are available in various qualities. Especially preferred are highly purified qualities such as Carbovax™ Sentry™ mPEG350 from The Dow Chemical Company.

Preferably, the polymers are methoxy-polyethyleneglycol (mPEG) and/or polyethylene glycols (PEG), having an average molecular weight ranging from 200 to 7500 or propylene glycol (PG) or mixtures thereof or single ethylene glycols such as tetraethylene glycol (4EG) and pentaethylene glycol (5EG).

According to a preferred aspect of the invention such as but not limited to vaginal or rectal delivery, the composition comprises less than 99% (w/w) of polyethylene glycol having an average molecular weight ranging from 200 to 7500.

In one aspect, the invention provides an antimicrobial food product comprising an antimicrobiologically active lipid as defined herein, in a concentration in the range from 0.01 to 5%, dissolved or suspended, ethanol in a concentration in the range from 0.1 to 4%, formulated in chocolate base to be administered in the oral cavity.

Accordingly, the active lipid ingredient and ethanol is in some embodiments added to chocolate base, such as but not limited to milk chocolate or dark chocolate to provide a solid formulation to be administered to the oral cavity, in order to achieve antimicrobial effects in such product. A non-limiting example of such formulation is exemplified in Example 3. Such formulations may comprise the active lipid component in a concentration such as mentioned above and preferably within a range of 0,1 to 1% and more preferably a range from 0,1% to 0,5%, ethanol in the above mentioned ranges, such as 1%, 1,5%, 2%, 2,25% or 2,5% ethanol, and 90-95% conventional chocolate, and some embodiments the remainder of the formulation is made up of the chocolate base.

The invention also relates to a method for treatment of animals such as pets: for example but not limited to dogs, cats, rabbits, guinea pigs, farm animals: such as but not limited to horses, sheep, pigs, cattle, chicken or captured wild animals with an effective amount of a biologically active lipid and ethanol, wherein the dosage unit quantity of a biologically active substance together with ethanol is applied to a surface of the animal to be treated in a formulation according to the invention. The volume administered to each animal, administration site, should preferably be calculated based on the relative human/animal surface area that need to be exposed.

The invention also relates to a method for treating industrial surfaces, walls, tables, floors, equipments, instruments or as an aerosol where bacteria, virus, fungi or prions may need to be eliminated.

The mucosal membrane to which the pharmaceutical preparation of the invention is administered may be any mucosal membrane of the mammal to which the biologically active lipid is to be given, e.g. in the nose, sinuses, vagina, eye, ear, mouth, oral cavity, pharynx, genital tract, lungs, gastrointestinal tract, or rectum, preferably the mucosa of the nose, sinuses mouth (buccal, gingual, sublingual or to the hard palate), pharynx, larynx, vagina, uterus and the air. The pharmaceutical preparation may also be administered to the skin and the nails.

The pharmaceutical compositions of the invention may be administered in the form of a sublingual lozenge or troche or a buccal, pharynx, ear, sinus or nasal spray or drops in the form of a solution, micells, nanoemulsion, optionally in water and/or together with plymers such as polyethylene glycol or propylene glycoloptionally in the form of slightly viscous solution or as a solid or semisolids in the form of a suppository or vagitory.

In some embodiments the formulation of the invention additionally comprises a polyoxyethylene-glyceride having the formula (II): 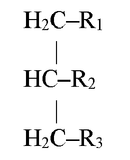 wherein R1, R2, and R3 are independently selected from the group consisting of C₆ to C₂₂ fatty acids (-OCO-C₅₋₂₁H₁₁₋₄₃), polyoxyethylene glycol (PEG) ((-O-CH₂-CH₂-)ₙ-H) polymer and hydrogen, provided that it contains at least one C₆-C₂₂ fatty acid and at least one PEG group.

The term polyoxyethylene-glyceride as used herein refers to a glyceride which is a mono- or diglyceride, i.e. with one or two fatty acid moieties connected to the glycerol backbone, and one or two polyoxyethylene glycol groups connected to one or both of the remaining one or two sites on the glycerol backbone of the glyceride. The set of substances referred to as polyoxyethylene-glyceride may also be referred to as polyoxyethylene glycol glycerides, polyoxyethylene mono- and diglycerides, or polyoxyethylene glycol mono- and diglycerides, PEG-glycerides or PEG mono- and diglycerides. Accordingy, the polyoxyethylene-glycerides used in the invention are suitably defined by Formula I and the definition provided above.

As mentioned above, the fatty acid component of the PEG-glyceride comprises C₆-C₂₂ fatty acid and preferably C₆-C₁₈ fatty acid, saturated or unsaturated, such as C₆-C₁₄ fatty acid, C₈ or C₁₀ fatty acid, or a combination thereof. Examples of C₆ to C₁₈ carboxylic acids, which are useful for the fatty acid R1, R2 or R3 component in formula (II) above are caproic, caprylic, capric, lauric, myristic, oleic, palmitic and stearic acid. Especially suitable for this invention are capric and caprylic acids, individually or together. In some embodiments a polyoxyethylene glycol glyceride is selected which is a combination product containing one or more substance(s) represented in the formula II being polyoxyethylene glycol (PEG)-fatty acid mono- or diglyceride such as macrogol-6-glycerol caprylocaprate, a mixture of mono and diesters made of polyoxyethyl glycerol ethers such as Softigen 767 from Cremer GmbH (Germany) or caprylocaproyl macrogol-8 glycerides, a mixture of mono-, di- and triglycerides and mono and di-fatty acid exters of polyethylene glycol such as Labrasol from Gattefosse (France).

The polyoxyethylene glycol (PEG or PEO) component used in the formation of the absorption promoter is, typically, a medium to high molecular weight material having a molecular weight of from about 200 to about 1200 such as, e.g., from about 300 to about 600. Suitable PEG-glycerides comprise in preferred embodiments a PEG component with a number of ethylene oxide units within the range from about 2, or from about 3, or from about 4, or from about 5 or from about 6, to about 30, such as to about 20, or to about 15, or to about 12, or to about 10, or to about 8, such as but not limited to having an average of about 5 ethylene oxide units, about 6 ethylene oxide units, about 7 ethylene oxide units, about 8 ethylene oxide units, or about 10 ethylene oxide units. For example, the above mentioned component magrogol-6-glycerol caprylocaprate is a mixture of mainly mono- and diesters of polyoxyethylene glycerol ethers mainly with caprylic (octanoic) and capric (decanoic) acids, with an average content of ethylene oxide being 6 units per molecule. Macrogol 6 glycerol caprylocaprate may be obtained by ethoxylation of glycerol and esterification with distilled coconut or palm kernel fatty acids, or by ethoxylation of mono- and diglycerides of caprylic and capric acids. Labrasol® is another example of a PEG-glyceride useful in the invention. Labrasol is defined by the manufacturer as Caprylocaproyl macrogol-8 glycerides, with the fatty acid component being mainly caprylic (octanoic) and caproic (hexanoic) acid with the PEG component having an average of about 8 ethylene oxide units.

The PEG glycerol is preferably present at a concentration within a range from 0,1%, such as from 0,2%, such as from 0,5%, more preferably from 1%, such as from 2%, such as from 4% or from 5%, to 60%, such as to 50%, such as to 40%, such as to 30% or to 25%, such as to 20%, such as to 15% or to 10%, such as but not limited to 2%, 5%, 7,5%, 10%, 12%, 15% or 20%.

Both the methoxypolyethylene glycol of the formula I and the polyoxyethylene glycol (PEG)-fatty acid mono- or diglyceride are considered to be a pharmaceutically acceptable carrier, especially a pharmaceutically acceptable carrier for mucosal and dermal (surface) administration. According to another aspect of the invention methoxypolyethylene glycols and polyoxyethylene glycol (PEG)-fatty acid mono- or diglyceride are mixed together with ethanol.

If desired, the pharmaceutical compositions of the present invention can optionally include additional compounds to enhance the solubility of the therapeutic agent. Examples of such compounds, include: alcohols and polyols, such as isopropanol, butanol, benzyl alcohol, ethylene glycol, propylene glycol, butanediols and isomers thereof, glycerol, pentaerythritol, sorbitol, mannitol, transcutol, dimethyl isosorbide, polyethylene glycol, polypropylene glycol, polyvinylalcohol, hydroxypropyl methylcellulose and other cellulose derivatives, cyclodextrins and cyclodextrin derivatives; ethers of polyethylene glycols having an average molecular weight of about 200 to about 6000 or tetrahydrofurfuryl alcohol PEG ether (glycofurol, available commercially from BASF under the trade name Tetraglycol); amides, such as 2-pyrrolidone, 2-piperidone, .epsilon.-caprolactam, N-alkylpyrrolidone, N-hydroxyalkylpyrrolidone, N-alkylpiperidone, N-alkylcaprolactam, dimethylacetamide, and polyvinylpyrrolidone; esters, such as ethyl propionate, tributylcitrate, acetyl triethylcitrate, acetyl tributyl citrate, triethylcitrate, ethyl oleate, ethyl caprylate, ethyl butyrate, triacetin, propylene glycol monoacetate, propylene glycol diacetate, .epsilon.-caprolactone and isomers thereof, delta.-valerolactone and isomers thereof, beta.-butyrolactone and isomers thereof; and other solubilizers known in the art, such as dimethyl acetamide, dimethyl isosorbide (Arlasolve DMI (ICI)), N-methyl pyrrolidones (Pharmasolve (ISP)), monooctanoin, diethylene glycol monoethyl ether (available from Gattefosse under the trade name Transcutol), and water.

For the manufacturing of suppositories or vagitories or in case there is a need for additional fat, the formulation may additionally contain one or more substance from cocoa butter, high molecular weight polyethylene glycol, castor oil, paraffin oil, and adeps solidus.

Mixtures of solubilizers are also within the scope of the invention. Except as indicated, these compounds are readily available from standard commercial sources.

Preferred solubilizers include triacetin, triethylcitrate, ethyl oleate, ethyl caprylate, dimethylacetamide, N-methylpyrrolidone, N-hydroxyethylpyrrolidone, polyvinylpyrrolidone, hydroxypropyl methylcellulose, hydroxypropyl cyclodextrins, ethanol, methoxy-polyethylene glycol 350-1500, polyethylene glycol 200-1000, glycofurol, transcutol, propylene glycol, and dimethyl isosorbide. Particularly preferred solubilizers include sorbitol, glycerol, triacetin, ethyl alcohol, mPEG 350-550, PEG-300-400, glycofurol and propylene glycol.

The amount of solubilizer that can be included in compositions of the present invention is not particularly limited. Of course, when such compositions are ultimately administered to a patient, the amount of a given solubilizer is limited to a bioacceptable amount, which is readily determined by one of skill in the art. In some circumstances, it may be advantageous to include amounts of solubilizers far in excess of bioacceptable amounts, for example, to maximize the concentration of therapeutic agent, with excess solubilizer removed prior to providing the composition to a patient using conventional techniques, such as distillation or evaporation. Typically, the solubilizer will be present in an amount of 1% to 100%, more typically 5% to 75% by weight or 5% to 25% by weight.

Other excipients that may be needed in the formulation are following: pH-controlling agents, such as, nitric acid, phosphoric acid, or acetic acid, citrate: Preservatives and osmotic pressure controlling agents, such as glycerol, sodium chloride, methyl paraoxybenzoate, or benzoic acid; Powder compositions, such as, alfa-, beta- and gamma-cyclodextrines, cellulose and derivatives; Microspheres,nanospheres, virosomes, proteosomes, liposomes and emulsions compositions, such as, starch, albumin, gelatine, or lecithins or lysoleciythins; Microencapsulated formulations; Propellants such as butane; Water.

Based on experiments presented in the Examples, it is presently preferred that the lipid is selected from capric acid 1-monoglyceride, lauric acid and/or mixtures thereof, as these have shown very high microbicidal activities with synergy together with ethanol. The presently most preferred lipid is capric acid 1-monoglyceride or lauric acid 1-monoglyceride together with ethanol according to the invention.

In the formulations used in the method of the present invention, the microbicidal lipid or lipids is/are preferably present in the formulation in a total concentration within a range from about 1 millimolar, such as from about 2 millimolar, or from about 5 millimolar, to about 40 millimolar, such as to about 30 millimolar, or to about 25 millimolar. preferably in a concentration of about 5 to 30 millimolar.

The formulation may also contain a spermicide such as but not limited to surfactants such as nonoxynol-9, chelating agents such as ethylenediaminetetraacetic acid (EDTA), channel-forming ionophores such as gramicidin, and other spermicidal agents such as benzalkonium chloride, sodium docusate and cholatc acid and salts thereof.

Examples of infections that can be treated or prevented by the formulations and method according to the invention may be any infection of the skin or mucosa caused by bacteria, virus or fungi towards which the microcidal lipids described herein are effective. Mucosa or mucosal membranes or surfaces may be the oral, aural, nasal, lung, gastro-intestinal, vaginal or rectal mucosa (as well as the surroundings) and the skin may be intact skin or skin which in some way have been injured. Examples of such fungi, bacteria and virus which can cause infection of the skin or mucosa are e.g. fungi such as e.g. Dermatophytes, Black piedra, White piedra, Tines nigra, and Tines versicolor; bacteria such as e.g. Escherichia coli, Pseudomonas aerginosa, and Staphylooccus aureus; virus such as e.g. influenza virus A, influenza virus B, influenza virus C, parainfluenza virus, mumps virus, Newcastle disease virus, viruses of rinderpest, canine distemper virus, respiratory syncytial virus, rabies virus, herpes simplex type 1, herpes simplex type 2, herpes genitalis, varicella zoster, cytomegalovirus, and Epstein-Barr virus.

It is also contemplated that the lipid is useful for the prevention or treatment of infection by a retrovirus such as e.g. human immuno deficiency Virus (HIV), sarcoma viruses, leukemia viruses, and human lymphotropic viruses types 1 and 2, and/or for the prevention or treatment of acquired immune deficiency syndrome (AIDS).

### Examples

The following examples are provided to illustrate specific working embodiments of the invention without limiting its scope.

### EXAMPLE 1

Formulations were made to be used as nasal, pharynx, larynx and sinus spray or ear drops to prevent and/or fight infections in the nose, pharynx, larynx, the sinuses, ear canals, external ear etc. with the following composition:

**Formulation**

| ***Component*** | ***I*** | ***II*** | ***III*** | ***IV*** |
|---|---|---|---|---|
| Monocaprin | 0.5% | 0.15% | - | 0.15 |
| Monolaurin | - | 0.35% | 0.5% | 0.35 |
| mPEG* | 96.5% | 96.5% | 96.5% | 99.5 |
| Ethanol | 3.0% | 3.0% | 3.0% | - |

| | | | | |
|---|---|---|---|---|
| *Methoxypolyethyelene glycol 350 | | | | |

Here the monocaprin and monolaurin are dissolved in the mPEG using vortex, ultrasound, heat and/or other standard methods, followed by addition of ethanol whereafter the mixture is mixed well together using standard methods to receive a homogeneous transparent solution.

Antimicrobial test showed that formulations I, II and III were able to induce 100% killing of *Pseudomonas aeruginosa, Staphylococcus aureus* as well as *Streptococcus pneumoniae* and *Haemopilus influenza.* Formualtion IV however, was only able to kill S. *pneumoniae* and *H. influenza.*

### EXAMPLE 2

Formulations were made to be used as nasal spray or ear drops to prevent and/or fight infections in the nose, the sinuses, ear canals, external ear etc. according to the invention may contain following formulation:

**Formulation**

| ***Component*** | ***I*** | ***II*** |
|---|---|---|
| Monocaprin | 0.1-0.5% | 0.1-0.5% |
| Monolaurin | 0.1-0.5% | 0.1-0.5% |
| mPEG* | 2% | 2% |
| Propylene glycol | 4% | 4% |
| Labrasol or Softigen 767 | 10% | 10% |
| Ethanol | - | 2% |
| Polysorbate 80 | 0,8% | 0,8% |
| Water | 82,2% | 80,2% |

| | | |
|---|---|---|
| *Methoxypolyethylene glycol 350 | | |

Here the mPEG, propylene glycol, polysorbate 80, ethanol (for formulation II) and either Labrasol or Softigen 767 are mixed together whereafter monocaprin and monolaurin are dissolved in this mixture using vortex, ultrasound, heat and/or other standard methods. Then water is added to the mixture and gently mixed together using standard methods to receive a homogeneous transparent solution.

Antimicrobial test showed that formulation II was more effective in killing *Pseudomonas aeruginosa* and *Staphylococcus aureus* than formulation I.

### EXAMPLE 3

Formulations were made to be melted in the buccal cavity (the mouth), to fight infections or fungi in the oral cavity e.g. due to denture, according to the invention may contain following formulation:

**Formulation**

| ***Component*** | ***I*** | ***II*** |
|---|---|---|
| Monocaprin | 0,15% | 0,25% |
| Monolaurine | 0,35% | 0,25% |
| Ethanol | 2% | 2% |
| Dark chocolate base | 97.5% | 97.5% |

Here the monocaprin, monolaurin and ethanol dissolved in the chocolate base using mild heat and/or other standard methods to receive a homogeneous mass.

### EXAMPLE 4

Formulations were made that can be used as a nasal spray and eardrops, to prevent and/or fight infections in nose, sinuses, larynx, pharynx, eye, oral cavity, ear canals, external ear etc., according to Examples 1, 2 and 3. However, instead of a mixture of monocaprin 0.15-0.5% and monolaurin 0.15-0.5%, there is only monocaprin in 0.5-1% or only monolaurine 0.5-1%.

### EXAMPLE 5

Formulations according to the invention were tested towards three bacterial strains: *Staphylococcus aureus* (ATCC 25923), *Streptococcus pneumoniae* (ATCC 49619), *Haemophilus influenza* (NCTC 8468). The bacteria were distributed in a thin layer across a petri dish with agar. Two plates were wetted with 20 µl of each formulation. The bacteria were allowed to grow at 37°C for 24 hours. *H. influenzae* and *S. pneumoniae* were grown under CO₂, but *S. aureus* was grown in normal atmosphere.
Readings of results were carried out 24 hours later.

The following formulations were tested:
Control: Methoxy-polyethyleneglycol (mPEG) 95% and ethanol 5%.
Test 1: Monocaprin 0.15%, monolaurate 0.35%, mPEG 94.5% and ethanol 5%
Test 2: Monocaprin 0.15%, monolaurate 0.35%, mPEG 95.5% and ethanol 4%
Test 3: Monocaprin 0.15%, monolaurate 0.35%, mPEG 97.5% and ethanol 2%
Test 4: Monocaprin 0.15%, monolaurate 0.35%, mPEG 98.5% and ethanol 1%

### Results:

There was an intensive growth in the agar plates, containing control. However for Test 1, 2, 3 and 4 gave following results:
*Staphylococcus aureus* = 17 mm; 19 mm; 18.9 mm; and 15.3 mm respectively.
*Streptococcus pneumoniae* = 7.9 mm; 8.4 mm; 15.4 mm; and 14.1 mm respectively.
*Haemophilus influenza* = 8.0 mm; 8.5 mm; 7.2 mm; and 11.0 mm respectively.

This illustrates that ethanol in a concentration similar or slightly higher than what is used in the invention has essentially no effect against the tested bacteria.

As used herein, including in the claims, singular forms of terms are to be construed as also including the plural form and vice versa, unless the context indicates otherwise. Thus, it should be noted that as used herein, the singular forms "a," "an," and "the" include plural references unless the context clearly dictates otherwise.

Throughout the description and claims, the terms "comprise", "including", "having", and "contain" and their variations should be understood as meaning "including but not limited to", and are not intended to exclude other components.

Use of exemplary language, such as "for instance", "such as", "for example" and the like, is merely intended to better illustrate the invention and does not indicate a limitation on the scope of the invention unless so claimed. Any steps described in the specification may be performed in any order or simultaneously, unless the context clearly indicates otherwise.

## Claims

1. A pharmaceutical antimicrobial liquid formulation comprising:
a. an antimicrobiologically active lipid in a concentration in the range from 0.01 to 5% selected from the group consisting of glycerol monocaprate, glycerol monocaprylate, glycerol monolaurate, propylene glycol monocaprate, propylene glycol monocaprylate, propylene glycol monolaurate, glycerol dicaprin, glycerol dicaprylate, glycerol dilaurate, glycerol tricaprin, glycerol tricaprylate, and glycerol trilaurate, and any combination thereof, dissolved or suspended in the formulation,
b. ethanol in a concentration in the range from 0.2 to 4%, and
c. a physiologically acceptable vehicle.

2. The pharmaceutical antimicrobial liquid formulation according to claim 1,
wherein the antimicrobiologically active lipid is in a concentration in the range from 0.05% to 2%, more preferably in the range from 0.1% to 1%.

3. The pharmaceutical antimicrobial liquid formulation according to any of the preceding claims, in a form selected from the group consisting of solution, ointment, spray, aerosol, mist, drops, creme, gel, suppository, and vagitory.

4. The pharmaceutical antimicrobial liquid formulation according to any of the preceding claims, further comprising methoxy-polyethylene glycol.

5. The pharmaceutical antimicrobial liquid formulation according to claim 4, wherein the methoxypolyethylene glycol is represented by formula I:
**CH**₃-(O-CH₂-CH₂)ₙ-H (I)
wherein n is an integer in the range from 1 to 25.

6. The pharmaceutical antimicrobial liquid formulation according to claim 4 or 5, wherein said methoxypolyethylene glycol is in a concentration within the range from 0.1% to 60%.

7. The pharmaceutical antimicrobial formulation according to any of the preceding claims, further comprising one or more excipient selected from the group consisting of absorption promoters, water absorbing polymers, microspheres, oils, emulsions, liposomes, substances that inhibit enzymatic degradation, alcohols, organic solvents, water, surfactants, hydrophobic agents, pH-controlling agents, preservatives and osmotic pressure controlling agents, cyclodextrines and propellants or mixtures thereof.

8. The pharmaceutical antimicrobial formulation according to any of the preceding claims, further comprising a substance selected from polyethylene glycol, a buffer, a spermicide such as nonoxynol-9, a chelating agent, including EDTA.

9. The pharmaceutical antimicrobial liquid formulation according to any of the preceding claims, formulated for administration selected from topical administration to skin, administration to oral cavity, administration to nasal mucosa, ocular administration, otal administration, administration pharynx, administration larynx, administration sinuses, vaginal administration, rectal administration, and topical administration to nails.

10. The pharmaceutical antimicrobial liquid formulation according to any of the preceding claims, provided in a dosage unit of less than or about 10 mL, preferably less than 1,0 mL, more preferably less than 500 µL and even more preferably in a volume within the range from 50 to 300 µL.

11. An antimicrobial food product comprising:
a. a an antimicrobiologically active lipid in a concentration in the range from 0.01 to 5% selected from the group consisting of glycerol monocaprate, glycerol monocaprylate, glycerol monolaurate, propylene glycol monocaprate, propylene glycol monocaprylate, propylene glycol monolaurate, glycerol dicaprin, glycerol dicaprylate, glycerol dilaurate, glycerol tricaprin, glycerol tricaprylate, and glycerol trilaurate, and any combination thereof, , dissolved or suspended in the formulation,
b. ethanol in a concentration in the range from 0.2 to 4%, formulated in chocolate base to be administered in the oral cavity.

12. The antimicrobial food product of claim 11, further comprising methoxy-polyethylene glycol.

13. An antimicrobiologically active lipid selected from the group consisting of glycerol monocaprate, glycerol monocaprylate, glycerol monolaurate, propylene glycol monocaprate, propylene glycol monocaprylate, propylene glycol monolaurate, glycerol dicaprin, glycerol dicaprylate, glycerol dilaurate, glycerol tricaprin, glycerol tricaprylate, and glycerol trilaurate, and any combination thereof, for use as a medicament for the treatment of a disease or condition of a mammal selected fom infections in the nasal, ocular, otal, pharynx, larynx, sinuses, oral cavity, vaginal or dermal surface of a mammal, caused by virus, pathogenic bacteria or fungi, wherein the lipid is formulated in a formulation comprising in the range from 0.01% to 5% of said lipid, ethanol in a concentration in the range from 0.2% to 4%, in physiologically acceptable vehicle.

14. An antimicrobiologically active lipid for use according to claim 13, wherein the mammal is a human.

15. A method to augment the microbicidal or antimicrobial effects of antimicrobiological lipid selected from the group consisting of glycerol monocaprate, glycerol monocaprylate, glycerol monolaurate, propylene glycol monocaprate, propylene glycol monocaprylate, propylene glycol monolaurate, glycerol dicaprin, glycerol dicaprylate, glycerol dilaurate, glycerol tricaprin, glycerol tricaprylate, and glycerol trilaurate, and any combination thereof, , in a pharmaceutical formulation, comprising admixing in the formulation ethanol in a concentration within the range of 0.2-4%, and a physiologically acceptable vehicle.

## Patentansprüche

1. Pharmazeutische antimikrobielle flüssige Formulierung, umfassend:
a. ein antimikrobiologisch aktives Lipid in einer Konzentration im Bereich von 0,01 bis 5 % ausgewählt aus der Gruppe bestehend aus Glycerinmonocaprat, Glycerinmonocaprylat, Glycerinmonolaurat, Propylenglykolmonocaprat, Propylenglykolmonocaprylat, Propylenglykolmonolaurat, Glycerindicaprin, Glycerindicaprylat, Glycerindilaurat, Glycerintricaprin, Glycerintricaprylat und Glycerintrilaurat und jeglicher Kombination davon, gelöst oder suspendiert in der Formulierung,
b. Ethanol in einer Konzentration im Bereich von 0,2 bis 4 %, und
c. ein physiologisch akzeptables Vehikel.

2. Pharmazeutische antimikrobielle flüssige Formulierung nach Anspruch 1, wobei das antimikrobiologisch aktive Lipid in einer Konzentration im Bereich von 0,05 % bis 2 %, bevorzugter im Bereich von 0,1 % bis 1 % vorhanden ist.

3. Pharmazeutische antimikrobielle flüssige Formulierung nach einem der vorhergehenden Ansprüche in einer Form ausgewählt aus der Gruppe bestehend aus Lösung, Salbe, Spray, Aerosol, Nebel, Tröpfchen, Creme, Gel, Zäpfchen und Vaginalzäpfchen.

4. Pharmazeutische antimikrobielle flüssige Formulierung nach einem der vorhergehenden Ansprüche, des Weiteren umfassend Methoxypolyethylenglykol.

5. Pharmazeutische antimikrobielle flüssige Formulierung nach Anspruch 4, wobei das Methoxypolyethylenglykol durch Formel I wiedergegeben wird:
CH₃-(O-CH₂-CH₂)ₙ-H (I)
wobei n für eine ganze Zahl im Bereich von 1 bis 25 steht.

6. Pharmazeutische antimikrobielle flüssige Formulierung nach Anspruch 4 oder 5, wobei das Methoxypolyethylenglykol in einer Konzentration im Bereich von 0,1 % bis 60 % vorliegt.

7. Pharmazeutische antimikrobielle Formulierung nach einem der vorhergehenden Ansprüche, des Weiteren umfassend einen oder mehrere Hilfsstoffe ausgewählt aus der Gruppe bestehend aus Absorptionspromotoren, Wasser absorbierenden Polymeren, Mikrosphären, Ölen, Emulsionen, Liposomen, Substanzen, welche den enzymatischen Abbau hemmen, Alkoholen, organischen Lösungsmitteln, Wasser, Tensiden, hydrophoben Mitteln, pH-Wertsteuerungsmitteln, Konservierungsmitteln und Steuerungsmitteln des osmotischen Drucks, Cyclodextrinen und Treibmitteln oder Mischungen davon.

8. Pharmazeutische antimikrobielle Formulierung nach einem der vorhergehenden Ansprüche, des Weiteren umfassend eine Substanz ausgewählt aus Polyethylenglykol, einem Puffer, einem Spermizid, wie Nonoxynol-9, einem Chelatbildner, einschließlich EDTA.

9. Pharmazeutische antimikrobielle flüssige Formulierung nach einem der vorhergehenden Ansprüche, die zur Verabreichung ausgewählt aus topischer Verabreichung an die Haut, Verabreichung in die Mundhöhle, Verabreichung an die Nasenschleimhaut, okulare Verabreichung, otale Verabreichung, Verabreichung an den Pharynx, Verabreichung an den Larynx, Verabreichung an die Nebenhöhlen, vaginale Verabreichung, rektale Verabreichung und topische Verabreichung an Nägel formuliert ist.

10. Pharmazeutische antimikrobielle flüssige Formulierung nach einem der vorhergehenden Ansprüche, die in einer Dosiseinheit von weniger als oder etwa 10 mL, vorzugsweise weniger als 1,0 mL, bevorzugter weniger als 500 µL und noch bevorzugter in einem Volumen innerhalb des Bereichs von 50 bis 300 µL bereitgestellt wird.

11. Antimikrobielles Nahrungsprodukt, umfassend:
a. ein antimikrobiologisch aktives Lipid in einer Konzentration im Bereich von 0,01 bis 5 % ausgewählt aus der Gruppe bestehend aus Glycerinmonocaprat, Glycerinmonocaprylat, Glycerinmonolaurat, Propylenglykolmonocaprat, Propylenglykolmonocaprylat, Propylenglykolmonolaurat, Glycerindicaprin, Glycerindicaprylat, Glycerindilaurat, Glycerintricaprin, Glycerintricaprylat und Glycerintrilaurat und jeglicher Kombination davon, gelöst oder suspendiert in der Formulierung,
b. Ethanol in einer Konzentration im Bereich von 0,2 bis 4 %,
formuliert in einer Schokoladenbasis, die in die Mundhöhle zu verabreichen ist.

12. Antimikrobielles Nahrungsprodukt nach Anspruch 11, das des Weiteren Methoxypolyethylenglykol umfasst.

13. Antimikrobiologisch aktives Lipid ausgewählt aus der Gruppe bestehend aus Glycerinmonocaprat, Glycerinmonocaprylat, Glycerinmonolaurat, Propylenglykolmonocaprat, Propylenglykolmonocaprylat, Propylenglykolmonolaurat, Glycerindicaprin, Glycerindicaprylat, Glycerindilaurat, Glycerintricaprin, Glycerintricaprylat und Glycerintrilaurat und jeglicher Kombination davon zur Verwendung als Medikament zur Behandlung einer Erkrankung oder eines Zustands eines Säugers ausgewählt aus Infektionen in Nase, Augen, Ohren, Pharynx, Larynx, Nebenhöhlen, Mundhöhle, Vaginal- oder Hautoberfläche eines Säugers, die durch Viren, pathogene Bakterien oder Pilze hervorgerufen werden, wobei das Lipid in einer Formulierung formuliert ist, die im Bereich von 0,01 % bis 5 % des Lipids, Ethanol in einer Konzentration im Bereich von 0,2 % bis 4 % in physiologisch akzeptablen Vehikel umfasst.

14. Antimikrobiell aktives Lipid zur Verwendung nach Anspruch 13, wobei der Säuger ein Mensch ist.

15. Verfahren zur Verstärkung der mikrobiziden oder antimikrobiellen Wirkungen von antimikrobiologischem Lipid ausgewählt aus der Gruppe bestehend aus Glycerinmonocaprat, Glycerinmonocaprylat, Glycerinmonolaurat, Propylenglykolmonocaprat, Propylenglykolmonocaprylat, Propylenglykolmonolaurat, Glycerindicaprin, Glycerindicaprylat, Glycerindilaurat, Glycerintricaprin, Glycerintricaprylat und Glycerintrilaurat und jeglicher Kombination davon in einer pharmazeutischen Formulierung, umfassend Mischen von Ethanol in einer Konzentration im Bereich von 0,2 bis 4 % und eines physiologisch akzeptablen Vehikels in die Formulierung.

## Revendications

1. Formulation pharmaceutique antimicrobienne liquide comprenant :
a. un lipide antimicrobiologiquement actif en une concentration dans la plage de 0,01 à 5 % choisi dans le groupe constitué par le monocaprate de glycérol, le monocaprylate de glycérol, le monolaurate de glycérol, le monocaprate de propylèneglycol, le monocaprylate de propylèneglycol, le monolaurate de propylèneglycol, le dicaprate de glycérol, le dicaprylate de glycérol, le dilaurate de glycérol, le tricaprate de glycérol, le tricaprylate de glycérol et le trilaurate de glycérol, et une quelconque association de ceux-ci, dissous ou en suspension dans la formulation,
b. de l'éthanol en une concentration dans la plage de 0,2 à 4 % et
c. un véhicule physiologiquement acceptable.

2. Formulation pharmaceutique antimicrobienne liquide selon la revendication 1, dans laquelle le lipide antimicrobiologiquement actif est en une concentration dans la plage de 0,05 % à 2 %, plus préférablement dans la plage de 0,1 % à 1 %.

3. Formulation pharmaceutique antimicrobienne liquide selon l'une quelconque des revendications précédentes, sous une forme choisie dans le groupe constitué par une solution, une pommade, un spray, un aérosol, de fines gouttelettes vaporisées, des gouttes, une crème, un gel, un suppositoire et un ovule vaginal.

4. Formulation pharmaceutique antimicrobienne liquide selon l'une quelconque des revendications précédentes, comprenant en outre du méthoxypolyéthylèneglycol.

5. Formulation pharmaceutique antimicrobienne liquide selon la revendication 4, dans laquelle le méthoxypolyéthylèneglycol est représenté par la formule I :
CH₃-(O-CH₂CH₂)ₙ-H (I)
dans laquelle n est un nombre entier dans la plage de 1 à 25.

6. Formulation pharmaceutique antimicrobienne liquide selon la revendication 4 ou 5, dans laquelle ledit méthoxypolyéthylèneglycol est en une concentration à l'intérieur de la plage de 0,1 % à 60 %.

7. Formulation pharmaceutique antimicrobienne selon l'une quelconque des revendications précédentes, comprenant en outre un ou plusieurs excipients choisis dans le groupe constitué par les promoteurs d'absorption, les polymères absorbant l'eau, les microsphères, les huiles, les émulsions, les liposomes, les substances qui inhibent la dégradation enzymatique, les alcools, les solvants organiques, l'eau, les tensioactifs, les agents hydrophobes, les agents d'ajustement du pH, les conservateurs et les agents d'ajustement de la pression osmotique, les cyclodextrines et les propulseurs, ou des mélanges de ceux-ci.

8. Formulation pharmaceutique antimicrobienne selon l'une quelconque des revendications précédentes, comprenant en outre une substance choisie entre du polyéthylèneglycol, un tampon, un spermicide tel que le nonoxynol-9, un agent chélatant, notamment l'EDTA.

9. Formulation pharmaceutique antimicrobienne liquide selon l'une quelconque des revendications précédentes, formulée pour une administration choisie entre l'administration topique à la peau, l'administration à la cavité buccale, l'administration à la muqueuse nasale, l'administration oculaire, l'administration auriculaire, l'administration au pharynx, l'administration au larynx, l'administration aux sinus, l'administration vaginale, l'administration rectale et l'administration topique aux ongles.

10. Formulation pharmaceutique antimicrobienne liquide selon l'une quelconque des revendications précédentes, fournie en une dose unitaire d'environ 10 ml ou moins, de préférence de moins de 1,0 ml, plus préférablement de moins de 500 µl et plus préférablement encore en un volume à l'intérieur de la plage de 50 à 300 µl.

11. Produit alimentaire antimicrobien comprenant :
a. un lipide antimicrobiologiquement actif en une concentration dans la plage de 0,01 à 5 % choisi dans le groupe constitué par le monocaprate de glycérol, le monocaprylate de glycérol, le monolaurate de glycérol, le monocaprate de propylèneglycol, le monocaprylate de propylèneglycol, le monolaurate de propylèneglycol, le dicaprate de glycérol, le dicaprylate de glycérol, le dilaurate de glycérol, le tricaprate de glycérol, le tricaprylate de glycérol et le trilaurate de glycérol, et une quelconque association de ceux-ci, dissous ou en suspension dans la formulation,
b. de l'éthanol en une concentration dans la plage de 0,2 à 4 %,
formulés dans une préparation pour chocolat à administrer dans la cavité buccale.

12. Produit alimentaire antimicrobien selon la revendication 11, comprenant en outre du méthoxypolyéthylèneglycol.

13. Lipide antimicrobiologiquement actif choisi dans le groupe constitué par le monocaprate de glycérol, le monocaprylate de glycérol, le monolaurate de glycérol, le monocaprate de propylèneglycol, le monocaprylate de propylèneglycol, le monolaurate de propylèneglycol, le dicaprate de glycérol, le dicaprylate de glycérol, le dilaurate de glycérol, le tricaprate de glycérol, le tricaprylate de glycérol et le trilaurate de glycérol, et une quelconque association de ceux-ci, destiné à être utilisé en tant que médicament pour le traitement d'une maladie ou affection d'un mammifère choisie parmi les infections sur la surface nasale, oculaire, auriculaire, du pharynx, du larynx, des sinus, de la cavité buccale, vaginale ou dermique d'un mammifère, provoquées par un virus, des bactéries pathogènes ou des champignons, le lipide étant formulé dans une formulation comprenant dans la plage de 0,01 % à 5 % dudit lipide, de l'éthanol en une concentration dans la plage de 0,2 % à 4 %, dans un véhicule physiologiquement acceptable.

14. Lipide antimicrobiologiquement actif destiné à être utilisé selon la revendication 13, le mammifère étant un être humain.

15. Procédé pour augmenter les effets microbicides ou antimicrobiens d'un lipide antimicrobiologique choisi dans le groupe constitué par le monocaprate de glycérol, le monocaprylate de glycérol, le monolaurate de glycérol, le monocaprate de propylèneglycol, le monocaprylate de propylèneglycol, le monolaurate de propylèneglycol, le dicaprate de glycérol, le dicaprylate de glycérol, le dilaurate de glycérol, le tricaprate de glycérol, le tricaprylate de glycérol et le trilaurate de glycérol, et une quelconque association de ceux-ci, dans une formulation pharmaceutique, comprenant le mélange dans la formulation d'éthanol en une concentration à l'intérieur de la plage de 0,2-4 % et d'un véhicule physiologiquement acceptable.
